# EUROPÄISCHE PATENTSCHRIFT

(11) **EP 1 156 997 B1**
(45) Veröffentlichungstag und Bekanntmachung des Hinweises auf die Patenterteilung: **28.04.2004**
(21) Anmeldenummer: 00909137.2
(22) Anmeldetag: 02.02.2000
(51) Int. Cl.: C07C 65/24, C07C 51/353, C07F 5/02, C07C 67/343, C07C 69/94

(54) **VERFAHREN ZUR HERSTELLUNG VON (1,1':4',1'')-TERPHENYLVEBINDUNGEN**
METHOD FOR PRODUCING (1,1':4'1'')-TERPHENYL COMPOUNDS
PROCEDE DE PRODUCTION DE COMPOSES (1,1':4',1'')-TERPHENYLE

(30) Priorität: 24.02.1999 DE 19907904
(43) Veröffentlichungstag der Anmeldung: 28.11.2001
(73) Patentinhaber: Clariant GmbH, 65929 Frankfurt am Main (DE)
(72) Erfinder: SCHERER, Stefan, D-64572 Büttelborn (DE); HABER, Steffen, D-76829 Landau/Pfalz (DE)
(86) Internationale Anmeldenummer: PCT/EP2000/000834
(87) Internationale Veröffentlichungsnummer: WO 2000/050375

(56) Entgegenhaltungen:
- EP-A- 0 637 624
- WO-A-94/25050
- US-A- 5 693 611

## Beschreibung

Die vorliegende Erfindung betrifft ein Verfahren zur Herstellung von [1,1':4',1"]-Terphenylverbindungen, die in 4"-Stellung substituiert sind.

4"-Alkoxyterphenyl-4-carbonsäuren, deren Alkoxygruppe einen Alkylrest mit mittlerer Kettenlänge enthält, dienen in Verbindung mit dem Echinocandin B Macrocyclus als Baustein für die Herstellung von Wirkstoffen mit antibiotischen, insbesondere antifungalen Eigenschaften.

Diese Wirkstoffe weisen ein neuartiges Wirkungsprinzip auf und sind deshalb von besonderem Interesse (WO 94/25050 und EP 0 561 639).

Aus der Gruppe der 4"-substituierten p-Terphenyle ist die 4"-n-Pentoxy [1,1':4',1"]-terphenyl-4-carbonsäure, die nach Kupplung mit dem Echinocandin B Macrocyclus zu einem Produkt mit hervorragenden Eigenschaften führt, hervorzuheben.

Die WO 94/25050 beschreibt ein mehrstufiges Verfahren zur Herstellung von 4"-n-Pentoxy[1,1':4',1"]terphenyl-4-carbonsäure (vgl. Seiten 28 und 29 Part A, Part B und Part C).

In einem ersten Schritt wird 4"-Brom-4-hydroxybiphenyl mit einem n-Pentylhalogenid zu dem entsprechenden 4'-Brom-4-n-pentoxybiphenyl umgesetzt. Das 4'-Brom-4-npentoxybiphenyl wird in einem zweiten Schritt mit n-Butyllithium bei -78°C umgesetzt, wobei durch Transmetallierung 4'-Lithium-4-n-pentoxybiphenyl gebildet wird, das in einem weiteren Schritt ebenfalls bei -78°C mit Borsäuretriisopropylester umgesetzt wird. Nach Hydrolyse und Aufarbeitung erhält man die 4'-n-Pentoxybiphenyl-4-boronsäure, die in weiteren Schritten mit 4-lodbenzoesäure nach einer Standard-Suzuki-Kupplung umgesetzt wird. Die 4"-n-Pentoxy[1,1':4',1"]-terphenyl-4-carbonsäure fällt als Rohprodukt an, das durch Chromatographie an Silicagel gereinigt wird.

Die Art der Synthese ist nachfolgend in vereinfachter Form schematisch wiedergegeben

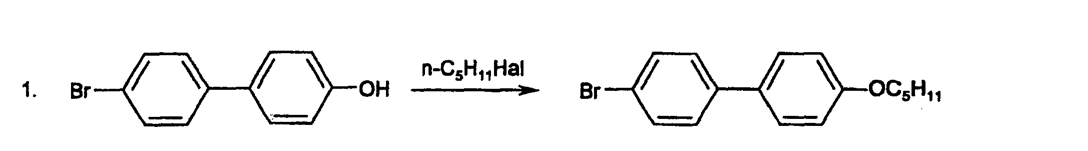

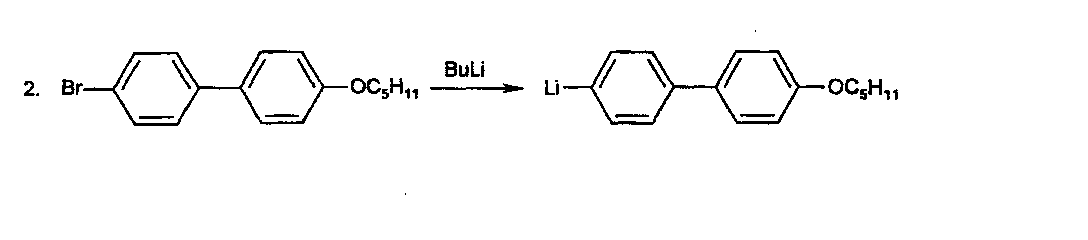

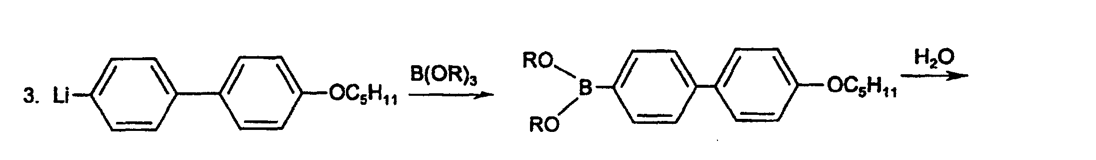

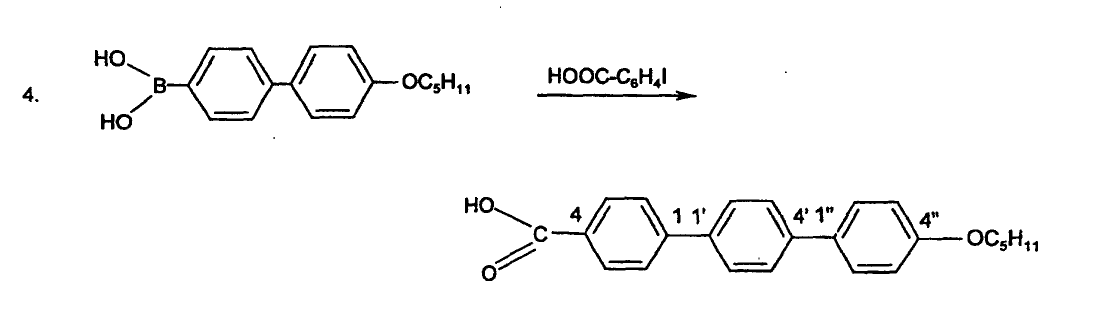

Die WO 94/25050 gibt lediglich für die Stufen (Part A und Part B) bis zur Bildung der 4-(4-n-Pentyloxyphenyl)phenylboronsäure Ausbeuten an. Aus Part C, der die Herstellung der 4"-n-Pentoxy[1,1':4',1"]terphenyl-4-carbonsäure betrifft, ist keine Ausbeuteangabe zu entnehmen.

Aus der EP 0 561 639 geht die Herstellung des 4"-n-Pentoxy[1,1':4',1"]terphenyl-4-carbonsäuremethylesters unter Anwendung der vorstehend erwähnten Reaktionsschritte 2, 3 und 4, wobei in Schritt 4 statt 4-lodbenzoesäure der 4-Iodbenzoesäurernethylester eingesetzt wird, hervor. Die Ausbeute beträgt für die 4'-n-Pentoxybiphenyl-4-boronsäure 44 % und für deren Umsetzung mit dem 4-lodbenzoesäuremethylester 64 % (vergl. Angaben Seite 26, Table 15 und 16 jeweils 2. Zeile quer), das heißt, die Gesamtausbeute beträgt lediglich 28,2 %, bezogen auf 4'-Brom-4-n-pentoxybiphenyl.

Das vorstehend beschriebene Verfahren weist mehrere Nachteile auf. Zum einen ist es erforderlich, von einem sehr reinen 4'-Brom-4-hydroxybiphenyl, das möglichst wenig Br-Stellungsisomere enthalten soll, auszugehen, um die geforderte Isomerenqualität im Endprodukt zu erfüllen. Zum anderen ist die Transmetallierung gemäß Schritt 2 recht aufwendig, da sie bei sehr tiefen Temperaturen durchzuführen ist. Wird diese Reaktion nicht in einem bestimmten Temperaturbereich gehalten und/oder sind die Reaktionszeiten zu lang, so entsteht infolge von Dimerisierung das entsprechende 4,4'''-Di-n-pentoxy[1,1':4',1":4",1''']quaterphenyl. Diese Verbindung läßt sich jedoch vom gewünschten Endprodukt nur mit großem Aufwand abtrennen. Auch die Umsetzung gemäß Schritt 3 wird bei sehr tiefer Temperatur durchgeführt. Nachteilig ist ferner, daß die 4"-Pentoxy[1,1':4',1"]terphenyl-4-carbonsäure als offensichtlich stark verunreinigtes Rohprodukt anfällt, das mittels Chromatographie an Silicagel gereinigt werden muß.

Im Hinblick hierauf besteht die Aufgabe, ein Verfahren bereitzustellen, das die vorstehend geschilderten Nachteile vermeidet und sich mit einem vertretbaren Aufwand durchführen läßt.

Diese Aufgabe wird gelöst durch ein Verfahren zur Herstellung von [1,1':4',1"]-Terphenylverbindungen der Formel 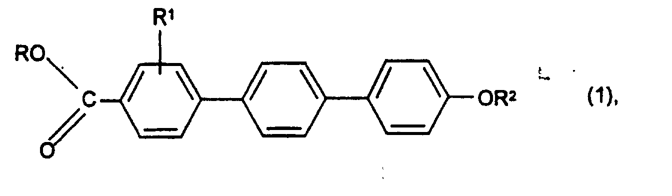 worin R Wasserstoff oder ein geradkettiger oder verzweigter (C₁-C₄)-Alkylrest, insbesondere Wasserstoff, ein (C₁-C₂)-Alkylrest oder C(CH₃)₃, R¹ Wasserstoff, ein geradkettiger oder verzweigter (C₁-C₄)-Alkylrest oder ein geradkettiger oder verzweigter (C₁-C₄)-Alkoxyrest, insbesondere Wasserstoff, ein (C₁-C₂)-Alkylrest oder (C₁-C₂)-Alkoxyrest, bevorzugt Wasserstoff, und R² Wasserstoff, ein geradkettiger (C₁-C₁₂)-Alkylrest, ein unsubstituierter Phenylrest, ein durch ein oder zwei (C₁-C₄)-Alkylgruppen oder (C₁-C₄)-Alkoxygruppen substituierter Phenylrest oder ein Rest - (CH₂)ₓOR³, worin x für eine ganze Zahl von 1 bis 4 und R³ für einen geradkettigen oder verzweigten (C₁-C₄)-Alkylrest steht, insbesondere ein geradkettiger (C₁-C₈)-Alkylrest, ein unsubstituierter Phenylrest oder ein Rest -(CH₂)ₓOR³, worin x für eine ganze Zahl von 1 bis 4 und R³ für einen geradkettigen oder verzweigten (C₁-C₄)-Alkylrest steht, bevorzugt ein geradkettiger (C₁-C₆)-Alkylrest oder ein Rest -(CH₂)ₓOR³, worin x für eine ganze Zahl von 1 bis 2 und R³ für einen geradkettigen oder verzweigten (C₁-C₄)-Alkylrest steht, ist.

Es ist dadurch gekennzeichnet, daß man ein Metallaryl der Formel 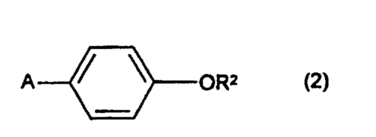 worin A für ein einwertiges Metall oder MeX, wobei Me ein zweiwertiges Metall und X Cl, Br oder I bedeutet, steht und R² für A oder einen trisubstituierten Silylrest steht oder die in Formel (1), ausgenommen Wasserstoff, angegebene Bedeutung hat, mit einem Borsäureester bei -80 bis 40°C, in Gegenwart eines inerten Lösungsmittels umsetzt, das Reaktionsprodukt durch Hydrolyse in eine Boronsäure der Formel 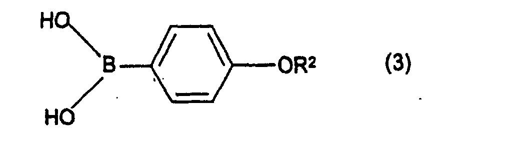 überführt, die Boronsäure, ein aus Boronsäure durch Abspalten von Wasser erhältliches Boronsäureanhydrid oder ein Gemisch aus Boronsäure und Boronsäureanhydrid mit einem Alkohol umsetzt, den dabei gebildeten Boronsäureester mit einer Biphenylverbindung der Formel 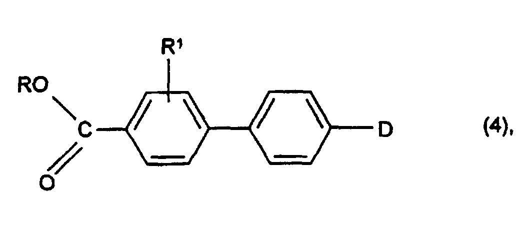 worin R und R¹ die in Formel (1) angegebene Bedeutung haben und D für Cl, Br, I, O₃S-CₙF₂ₙ₊₁, wobei n eine ganze Zahl von 1 bis 4 ist, oder N₂⁺Y⁻, wobei Y⁻ ClO₄⁻, BF₄⁻ oder HSO₄⁻ ist, steht, bei 40 bis 180°C in Gegenwart eines Katalysators und eines polaren Lösungsmittels umsetzt.

Das erfindungsgemäße Verfahren ist in vereinfachter Form nachfolgend schematisch wiedergegeben

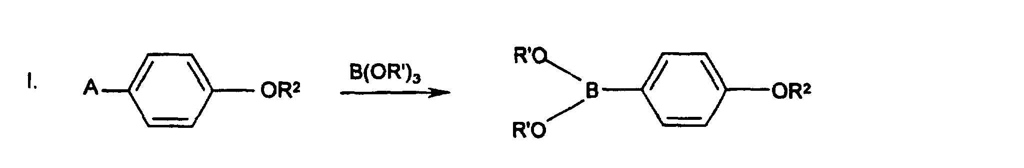

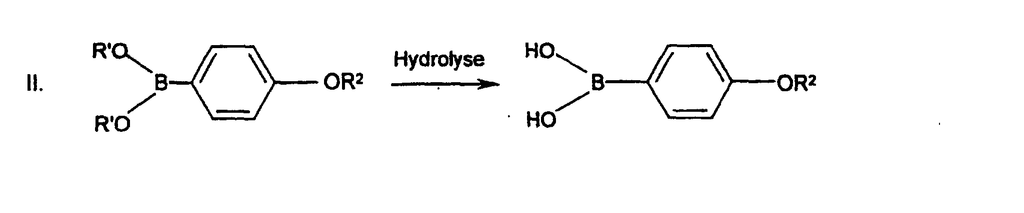

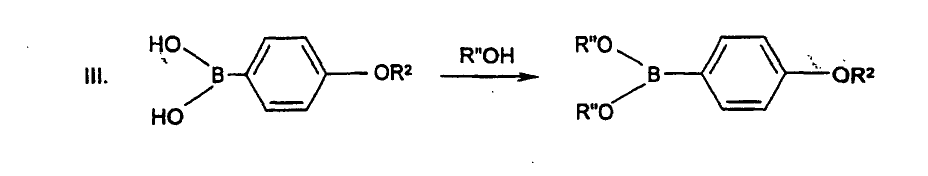

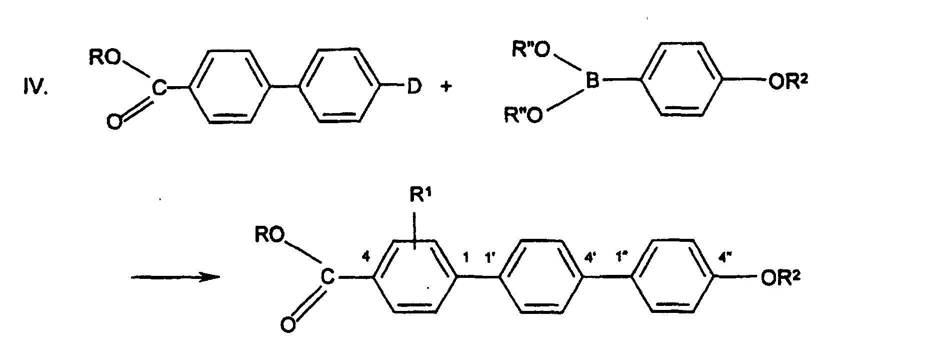

Das Metallaryl der Formel (2) kann man durch Umsetzung eines entsprechend in p-Stellung halogenierten Benzolderivats beispielsweise mit Mg oder einem Li-alkyl herstellen. Die Bildung einer Quaterphenylverbindung, die sich vom gewünschten Endprodukt nur schwer abtrennen läßt, findet nicht statt. Die Umsetzung des Metallaryls mit Borsäureester erfordert nicht in jedem Fall die in der WO 94/25050 angegebenen tiefen Temperaturen. Grignard-Verbindungen erlauben eine Umsetzung bei deutlich höheren Temperaturen als in der WO 94/25050 angegeben.

Man setzt üblicherweise ein Metallaryl der Formel (2), worin A für Li, Na, K, MgX oder ZnX, insbesondere für Li, MgX oder ZnX und X für Cl, Br oder I, insbesondere für Cl oder Br steht, ein.

Besonders einfach gestaltet sich das Verfahren, wenn man ein Metallaryl der Formel (2), worin A für MgCl, MgBr oder MgI, insbesondere für MgCl oder MgBr, bevorzugt für MgCl steht, einsetzt.

Wie zuvor bereits erwähnt, setzt man ein Metallaryl der Formel (2), worin R² für A oder einen trisubstituierten Silylrest steht oder die in der Verbindung der Formel (1) angegebene Bedeutung hat, hierbei jedoch nicht Wasserstoff sein kann, ein.

Beabsichtigt man eine Terphenylverbindung der Formel (1), worin R² Wasserstoff ist, herzustellen, so kann man von einem Metallaryl, (2), worin R² für A oder den trisubstituierten Silylrest steht, ausgehen und durch die nachfolgende Verarbeitung des Reaktionsproduktes die entsprechende phenolische Terphenylverbindung gewinnen.

Der trisubstituierte Silylrest im Metallaryl steht für einen Rest SiR⁴R⁵R⁶, worin die Reste R⁴, R⁵ und R⁶ gleich oder verschieden sind und einen Phenylrest oder einen (C₁-C₄)-Alkylrest, insbesondere gleich sind und einen (C₁-C₄)-Alkylrest, bedeuten. Der Silylrest fungiert als Schutzgruppe, die nach der Umsetzung leicht abgespalten werden kann, wobei die entsprechende Phenolgruppe gebildet wird. Ein besonders geeigneter trisubstituierter Silylrest ist der Si(CH₃)₃-Rest.

Man setzt einen Borsäureester B(OR')₃, worin R' gleich oder verschieden voneinander ist und für einen geradkettigen oder verzweigten (C₁-C₈)-Alkylrest, einen unsubstituierten oder durch eine oder zwei (C₁-C₄)-Alkylgruppen oder (C₁-C₄)-Alkoxygruppen substituierten Phenylrest, insbesonders für einen geradkettigen oder verzweigten (C₁-C₄)-Alkylrest, einen unsubstituierten oder durch ein oder zwei (C₁-C₄)-Alkylgruppen substituierten Phenylrest, bevorzugt für einen geradkettigen oder verzweigten (C₁-C₄)-Alkylrest oder einen unsubstituierten Phenylrest,besonders bevorzugt für einen geradkettigen oder verzweigten (C₁-C₄)-Alkylrest steht, ein.

Da die Borsäureester, deren Reste R' gleich sind, besonders gut zugänglich sind, setzt man in einer Vielzahl von Fällen Borsäureester der vorstehend genannten Art, deren Reste R' gleich sind, ein. Beispiele für derartige Borsäureester sind Borsäuretrimethylester, Borsäuretriethylester, Borsäuretri-n-propylester, Borsäuretriisopropylester, Borsäuretri-n-butylester und Borsäuretriisobutylester.

Man führt die Umsetzung des Metallaryls mit dem Borsäureester, wie eingangs bereits erwähnt, bei -80 bis +40°C, insbesondere -70 bis 10°C, bevorzugt -40 bis 0°C durch. Als inertes Lösungsmittel verwendet man beispielsweise einen Dialkylether mit 1 bis 4 C-Atomen je Alkylrest, einen cycloaliphatischen Ether mit 4 oder 5 C-Atomen im Ring, beispielsweise Tetrahydrofuran oder 1,4-Dioxan, ein Formaldehyd-dialkylacetal, einen 1,2-Dialkylglykoiether mit 1 bis 4 C-Atomen je Alkylrest, ein Gemisch derselben oder ein Gemisch derselben mit Toluol, insbesondere einen Dialkylether mit 1 bis 4 C-Atomen je Alkylrest, Tetrahydrofuran, ein 1,2-Dialkylglykolether mit 1 bis 4 C-Atomen je Alkylrest, ein Gemisch derselben oder ein Gemisch derselben mit Toluol, bevorzugt Tetrahydrofuran, Dibutylglykolether, Methyl-tert.-butylether, Diethylether, Diisopropylether,Di-n-butylether, ein Gemisch derselben oder ein Gemisch derselben mit Toluol.

Die Umsetzung des Borsäureesters mit dem Metallaryl führt zu einem salzartigen Additionsprodukt (Boratsalz). Nach Abschluß der Umsetzung zersetzt man das gegebenenfalls den Rest A oder den trisubstituierten Silylrest als Rest R² enthaltende Reaktionsprodukt und gegebenenfalls noch vorhandenes, nicht umgesetztes Metallaryl, indem man das Reaktionsgemisch mit Wasser oder einem Wasser-Eis-Gemisch zusammenbringt. Die Hydrolyse des Reaktionsproduktes verläuft ebenso wie die des Metallaryls sehr rasch ab, da sowohl das salzartige Additionsprodukt als auch das Metallaryl auch bei tiefen Temperaturen sehr schnell mit Wasser reagiert. Dabei bildet sich die Boronsäure (3) und es fallen Salze an, die auf das Reaktionsprodukt und gegebenenfalls auf noch vorhandenes ebenfalls hydrolysiertes Metallaryl zurückgehen.

Um Salze, insbesondere basische Salze, zu lösen, säuert man die erhaltene wäßrige Mischung, beispielsweise durch Zugabe einer Mineralsäure, insbesondere Salzsäure oder Schwefelsäure an. Es empfiehlt sich, um eine vollständige Auflösung der Salze sicherzustellen, einen pH-Wert von 0 bis 4, insbesondere 0,5 bis 3, bevorzugt 1 bis 2 einzustellen.

Anschließend führt man eine Phasentrennung durch und trennt die das inerte Lösungsmittel und die Boronsäure enthaltende, organische Phase ab. Falls gewünscht, kann man die Phasentrennung durch Zugabe eines geeigneten inerten Lösungsmittels, beispielsweise Ether, Methylenchlorid, Chloroform, Toluol, Chlorbenzol unterstützen.

Man versetzt die abgetrennte organische Phase mit Wasser, um gegebenenfalls noch vorhandene Salze zu lösen, und destilliert das inerte Lösungsmittel und das gegebenenfalls zur Unterstützung der Phasentrennung eingesetzte Lösungsmittel ab.

Die Boronsäure fällt hierbei als Feststoff aus. Man filtriert sie ab und trocknet sie. Führt man die Trocknung bei Temperaturen ≥ 30, insbesondere ≥ 50°C durch, so beginnt die Boronsäure unter Bildung des entsprechenden Anhydrids Wasser abzuspalten. Die Bildung der Boronsäureanhydride hängt einerseits von der Höhe der Temperatur und andererseits von der Zeit, während der die Temperatur auf die Boronsäure einwirkt, ab. Hohe Temperaturen und lange Einwirkungszeiten begünstigen die Boronsäureanhydridbildung.

Will man die Boronsäure erhalten, so empfiehlt es sich, die Trocknung bei niedrigen Temperaturen und unter Vakuum durchzuführen.

Es ist auch möglich, das Boronsäureanhydrid beispielsweise mittels einer wäßrigen Lauge zu hydrolysieren und die Boronsäure durch anschließendes Ansäuern der wäßrigen, Boronsäuresalz enthaltenden Lösung freizusetzen.

In einer Vielzahl von Fällen bildet sich ein Gemisch aus Boronsäure und Boronsäureanhydrid. Bei dem Boronsäureanhydrid handelt es sich um cyclische Anhydride, insbesondere um trimeres Boronsäureanhydrid. Es können sich unter Umständen möglicherweise auch Mischungen von Anhydriden bilden. Die Boronsäure, das Boronsäureanhydrid und das Gemisch aus Boronsäure und Boronsäureanhydrid lassen sich - falls gewünscht - durch Umkristallisieren in einem geeigneten Lösungsmittel, beispielsweise aliphatischen, cycloaliphatischen und/oder aromatischen Kohlenwasserstoffen, reinigen.

Im darauf folgenden Schritt wird die Boronsäure, das Boronsäureanhydrid oder das Boronsäure und Boronsäureanhydrid enthaltende Gemisch mit einem Alkohol umgesetzt. Diese Veresterung erfolgt nach gängigen Methoden. Ein Katalysator, beispielsweise eine Säure, muß nicht zugesetzt werden. Möglicherweise fungiert die Boronsäure, das Boronsäureanhydrid oder das Gemisch aus Boronsäure und Boronsäureanhydrid als Katalysator. Üblicherweise läßt man die Veresterung bei 50 bis 150°C, insbesondere 60 bis 140°C ablaufen.

Um die Umsetzung zu begünstigen, empfiehlt es sich, das sich infolge der Veresterung bildende Wasser zu entfernen. Dies kann beispielsweise durch azeotrope Destillation unter Auskreisen von Wasser oder durch Zusatz wasserentziehender Mittel, beispielsweise ortho-Ameisensäureester, geschehen. Geeignete Schleppmittel für die azeotrone Wasserabtrennung sind beispielsweise aliphatische oder aromatische Kohlenwasserstoffe, chlorierte aliphatische oder aromatische Kohlenwasserstoffe, Ether oder Ketone. Ohne Anspruch auf Vollständigkeit zu erheben seien als Schleppmittel Pentan, Hexan, Heptan, Cyclopentan, Cyclohexan, Toluol, Xylol, Ethylbenzol, Mesitylen, Dichlormethan, Chloroform, Tetrachlorkohlenstoff, Trichlorethylen, Chlorbenzol, Dichlorbenzol, Chlortoluol oder Dichlortoluol genannt

Als Alkohol setzt man einen (C₁-C₈)-Alkylalkohol, ein (C₂-C₆)-Alkandiol-1,2, ein (C₃-C₆)-Alkandiol-1,3, ein (C₄-C₆)-Alkandiol-1,4 oder 1,2-Dihydroxybenzol, insbesondere einen (C₁-C₈)-Alkylalkohol, ein (C₂-C₆)-Alkandiol-1,2 oder ein (C₃-C₆)-Alkandiol-1,3, bevorzugt einen (C₁-C₄)-Alkylalkohol, ein (C₂-C₄)-Alkandiol oder ein (C₃-C₅)-Alkandiol-1,3 ein.

Beispiele für Alkylalkohole sind Methanol, Ethanol, n-Propanol, i-Propanol, n-Butanol, i-Butanol, n-Pentanol, 2-Methylpentanol, n-Hexanol, 2-Ethylhexanol, insbesondere Methanol, Ethanol, n-Propanol, i-Propanol, n-Butanol und i-Butanol. Geeignete Alkandiole sind beispielsweise Ethylenglykol, Propandiol-1,3 und 2,2-Dimethylpropandiol-1,3 (Neopentylglykol).

Infolge der Umsetzung mit dem Alkohol bildet sich der entsprechende Boronsäureester, der anschließend mit der Biphenylverbindung der Formel (4) in Gegenwart eines Katalysators, eines säurebindenden Mittels und eines polaren Lösungsmittels umgesetzt wird.

Man kann aber anstelle des Boronsäureesters auch die Boronsäure der Formel (3), das aus der Boronsäure durch Abspalten von Wasser erhältliche Boronsäureanhydrid oder das Gemisch aus Boronsäure und Boronsäureanhydrid in diese Reaktion einsetzen und somit auf die Herstellung des Boronsäureesters durch Umsetzung der Boronsäure, des Boronsäureanhydrids oder des Gemisches aus Boronsäure und Boronsäureanhydrid mit dem Alkohol verzichten:

Die Umsetzung des Boronsäureesters, respektive der Boronsäure, des Boronsäureanhydrids oder des Gemisches aus Boronsäure und Boronsäureanhydrid erfolgt - wie bereits erwähnt - bei 40 bis 180°C, insbesondere 50 bis 130°C, bevorzugt 60 bis 120°C. Als säurebindende Mittel können Amine, beispielsweise aliphatische Amine, insbesondere Trialkylamine, basische Salze organischer und anorganischer Säuren, insbesondere Alkalisalze und Erdalkalisalze organischer und anorganischer Säuren, beispielsweise Na-acetat, K-acetat, Na₃PO₄, K₃PO₄, NaHCO₃, KHCO₃, Na₂CO₃, K₂CO₃, MgCO₃, CaCO₃, oder Alkalioxide, Alkalihydroxide, Erdalkalioxide, Erdalkalihydroxide, beispielsweise NaOH, KOH, Mg(OH)₂ oder Ca(OH)₂, dienen.

Als gut geeignete säurebindende Mittel haben sich AJkalihydrogencarbonate, Alkalicarbonate, Erdalkalihydrogencarbonate und Erdalkalicarbonate, insbesondere Na₂CO₃ und K₂CO₃, bevorzugt Na₂CO₃ erwiesen.
Als Biphenylverbindung der Formel 4 setzt man insbesondere diejenigen ein, in denen R für Wasserstoff oder einen geradkettigen oder verzweigten (C₁-C₄)-Alkylrest, insbesondere für Wasserstoff, einen (C₁-C₂)-Alkylrest oder C(CH₃)₃, bevorzugt für CH₃ oder C(CH₃)₃ steht, R¹ Wasserstoff oder ein geradkettiger oder verzweigter (C₁-C₄)-Alkylrest oder (C₁-C₄)-Alkoxyrest, insbesondere Wasserstoff, ein (C₁-C₂)-Alkylrest oder (C₁-C₂)-Alkoxyrest ist und D für Cl, Br, I oder N₂⁺Y⁻, insbesondere für Cl, Br oder I, bevorzugt für Br oder I steht.

Als polares Lösungsmittel kann ein protisches und aprotisches dipolares Lösungsmittel, insbesondere ein Alkohol, ein Sulfoxid, ein Sulfon, ein Amid und gegebenenfalls Wasser oder ein Gemisch derselben verwendet werden. Beispiele für Alkohole sind geradkettige oder verzweigte (C₁-C₄)-Alkylalkohole, Ethylenglykol, Polyethylenglykole der Formel HO-(CH₂-CH₂-O)ₙH mit n = 2 bis 1000 oder Gemische dieser Alkohole untereinander oder mit Wasser, insbesondere Ethylenglykol, Gemische von (C₁-C₄)-Alkylalkoholen mit Ethylenglykol oder Polyethylenglykolen oder mit Wasser, bevorzugt Gemische aus Methanol und Polyethylenglykolen, Methanol und Ethylenglykol oder Butanol und Wasser.

Beispiele für Sulfoxide sind Dimethylsulfoxid und Diethylsulfoxid.

Als Vertreter aus der Reihe der Sulfone ist Sulfolan (Thiolandioxid) und aus der Reihe der Amide sind Dimethylformamid, Diethylformamid, Dimethylacetamid, Diethylacetamid und N-Methylpyrrolidon zu nennen.

In einer Reihe von Fällen kann man auch Mischungen von Alkoholen, Sulfoxiden, Sulfolan und/oder Amiden, die gegebenenfalls auch Wasser enthalten können, einsetzen.

Als Katalysatoren eignen sich Palladium, eine Palladium- oder eine Nickeiverbindung. Man kann Pd-Metall, Pd(O)-Komplexverbindungen, Pd(II)-Komplexverbindungen, Ni(O)-Komplexverbindungen und Ni(II)-Komplexverbindungen, insbesondere Komplexverbindungen, die Phosphine, vorzugsweise trisubstituierte Phosphine wie Tri-n-butylphosphin, Tri-tert.-butylphosphin, Triphenylphosphin (PPh₃) enthalten, einsetzen.

Beispiele für Pd(O)-Kompfexverbindungen sind Pd(PPh₃)₄, Pd(dba)₂.

Beispiele für Pd(II)-Komplexverbindungen sind PdCl₂(PPh₃)₂, PdBr₂(PPh₃)₂, PdCl₂(R"CN)₂ PdBr₂(R"CN) mit R" = Phenyl, Methyl, PdCl₂(dppf), PdBr₂(dppf) mit dppf = 1,1'-Bis(diphenylphosphino)ferrocen, PdCl₂(COD), PdBr₂(COD) mit COD = Cycloocta-1,5-dien.

Beispiele für Ni(O)-Komplexverbindungen sind Ni(PPh₃)₄ und Beispiele für Ni(II)-Komplexverbindungen sind NiCl₂(PPh₃)₂, NiBr₂(PPh₃)₂, NiCl₂dppf und NiBr₂dppf.

Man kann auch Pd(II)-Verbindungen bzw. Ni(II)-Verbindungen, beispielsweise entsprechende Salze, zusammen mit den Phosphinen einsetzen. Hierbei bilden sich die entsprechenden Komplexverbindungen in situ.

Besonders geeignet sind Palladiumverbindungen, beispielsweise PdCl₂, Pd(acetat)₂.

Bei Herstellung von [1,1':4',1"]-Terphenyl-4-carbonsäuren (R = H in Formel (1)) empfiehlt es sich, das bei der Umsetzung der Biphenylverbindung (4) gebildete Reaktionsprodukt mit Wasser und einer Säure, insbesondere einer Mineralsäure, bevorzugt HCl oder H₂SO₄ zu behandeln, um die Hydrolyse der gebildeten Salze herbeizuführen oder zu vervollständigen. In einer Reihe von Fällen hat es sich bewährt, die Hydrolyse bei erhöhter Temperatur, beispielsweise bei 30 bis 100°C, insbesondere bei 60 bis 90°C durchzuführen.

Die vorliegende Erfindung betrifft ferner die Verbindungen 4-n-Pentoxyphenylboronsäure 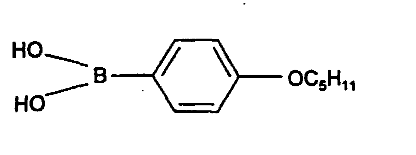 trimeres 4-n-Pentoxyphenylboronsäureanhydrid 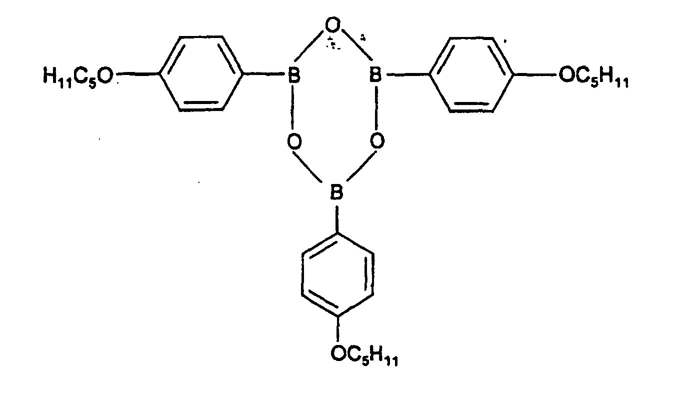 4-n-Pentoxyphenylboronsäure-glykolester 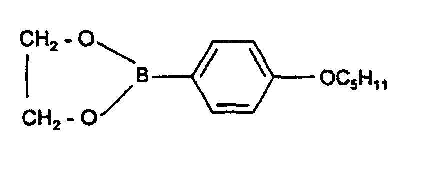 und 4-n-Pentoxyphenylboronsäure-neopentylglykolester

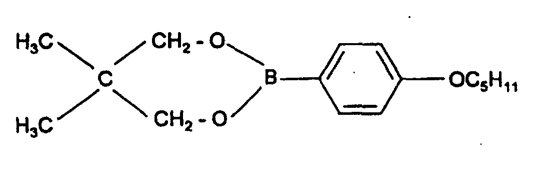

Die nachfolgenden Beispiele beschreiben die Erfindung näher, ohne sie zu beschränken.

### Experimenteller Teil

### Herstellung des Ausgangsmaterials

### Beispiel A

### Herstellung von 4-n-Pentoxyphenylmagnesiumchlorid (Ausgangsmaterial)

In einem Standardreaktionsglasgefäß werden unter Inertgasatmosphäre 134 g einer 30%igen Lösung von 4-Pentoxyphenylmagnesiumchlorid in Tetrahydrofuran zusammen mit 60,8 g Magnesiumspänen in 178 g Tetrahydrofuran vorgelegt und zum Sieden erhitzt. Man versetzt mit einem Zehntel einer Lösung von 497 g 4-Chlorphenylpentylether in 450 g Tetrahydrofuran. Nach Anspringen der Reaktion tropft man innerhalb von 5 Stunden die restliche Chloraromatenlösung zu. Ist die Zugabe beendet, wird weitere 3 Stunden unter Rückfluß erhitzt. Man verdünnt mit 675 g Tetrahydrofuran und filtriert bei Raumtemperatur von überschüssigem Magnesium unter Inertgasatmosphäre ab.
Man erhält 1930 g einer 30%igen Lösung von 4-Pentoxyphenylmagnesiumchlorid in Tetrahydrofuran.

### Herstellung von 4-n-Pentoxyphenylboronsäure und 4-n-Pentoxyphenylboronsäureanhydrid

### Beispiel 1

### Herstellung eines Gemisches von 4-n-Pentoxyphenylboronsäure und trimerem 4-n-Pentoxyphenylboronsäureanhydrid

In einem Standardreaktionsglasgefäß werden unter Inertgasatmosphäre 390 g Tetrahydrofuran zusammen mit 437 g Trimethylborat vorgelegt, auf -20°C gekühlt und mit 3000 g einer 29%igen Lösung von 4-Pentoxyphenylmagnesiumchlorid in Tetrahydrofuran so versetzt, daß die Innentemperatur -15°C nicht überschreitet. Die weiße Suspension wird nach beendeter Zugabe vorsichtig auf eine Mischung von 1135 g Wasser und 1135 g Eis gegeben und die erhaltene Mischung wird mit 325 g 60%iger Schwefelsäure auf pH 1-2 eingestellt. Nach Auflösen der Magnesiumsalze trennt man die Phasen, gibt die obere produkthaltige organische Phase (3200 g) auf 3 l Wasser und destilliert das Tetrahydrofuran weitgehend ab. Dabei fällt ein weißer Feststoff an. Man filtriert von der Boronsäure ab und trocknet diese bei 50°C/150 mbar. Dies liefert 540 g 4-Pentoxyphenylboronsäure als Gemisch mit dem trimeren Anhydrid, die zur weiteren Reinigung aus einem Kohlenwasserstoff (Hexan, Cyclohexan) umkristallisiert werden kann.

### Beispiel 2

### Herstellung von 4-n-Pentoxyphenylboronsäure

Aus dem ursprünglich erhaltenen Gemisch der Boronsäure mit dem trimeren Anhydrid erhält man die reine Boronsäure, indem man das Gemisch in überschüssiger Natronlauge in der Wärme löst und nach Abkühlen durch Zugabe von halbkonz. Salzsäure unter Eiskühlung die freie Boronsäure ausfällt. Nach Filtration und Waschen mit Wasser erhält man wasserfeuchte 4-n-Pentoxyphenylboronsäure vom Schmelzpunkt 75 - 80°C (auf Ton abgepreßt), die beim Trocknen zu einem gewissen Grad das trimere Anhydrid bildet (z. B. im Trockenschrank).
4-Pentoxyphenylboronsäure: IR-Spektrum
υ: 3334 (O-H), 2940, 1607, 1413, 1346, 1287, 1259, 1182, 1172, 1158, 1113, 1098, 1021, 997, 818 cm⁻¹.

### Beispiel 2a

### Herstellung von trimerem 4-n-Pentoxyphenylboronsäureanhydrid

Aus dem ursprünglich erhaltenen Gemisch der Boronsäure mit dem trimeren Anhydrid erhält man die reine Boronsäure, indem man entweder das Gemisch in Toluol azeotrop entwässert und das Anhydrid im Anschluß nach Entfemen des Toluols mit Cyclopentan fällt oder das Gemisch bei 50°C im Trockenschrank im Vakuum bis zur Gewichtskonstanz trocknet. Das trimere 4-n-Pentoxyphenylboronsäureanhydrid besitzt einen Schmelzpunkt von 102 - 103°C.
4-Pentoxyphenylboronsäureanhydrid: IR-Spektrum
u: 2932, 1604, 1414, 1381, 1368, 1356, 1346, 1305, 1292, 1270, 1247, 1173, 1021, 833 cm⁻¹.

### Herstellung von 4-n-Pentoxyphenylboronsäureestem

### Beispiel 3

### Herstellung von 4-n-Pentoxyphenylboronsäure-glykolester

250 g 4-n-Pentoxyphenylboronsäureanhydrid werden zusammen mit 81 g Ethylenglykol in 1000 ml Toluol in der Hitze gelöst. Das bei der Veresterung entstehende Wasser wird am Wasserabscheider ausgekreist. Nach beendeter Reaktion wird zunächst das Toluol abdestilliert und danach der Rückstand fraktionierend im Vakuum destilliert. Dies liefert 274 g 4-n-Pentoxyphenylboronsäureglykolester vom Sdp. 156°C/6 mbar, welcher nach einigen Stunden erstarrt (Schmelzpunkt 37-39°C).

### Beispiel 4

### Herstellung von 4-n-Pentoxyphenylboronsäure-neopentylglykolester

505 g 4-n-Pentoxyphenylboronsäureanhydrid werden zusammen mit 274 g 2,2-Dimethylpropan-1,3-diol in 2,5 l Toluol in der Hitze gelöst. Das bei der Veresterung entstehende Wasser wird am Wasserabscheider ausgekreist. Nach beendeter Reaktion wird das Toluol im Vakuum vollständig abdestilliert. Zum Rückstand werden 550 ml Cyclohexan gegeben, die Mischung wird bis zum Sieden erhitzt, filtriert und auf Raumtemp. gekühlt. Der ausgefallene 4-n-Pentoxyphenylboronsäure-(2,2-dimethyl-1,3-diol)ester wird abfiltriert und bei Raumtemperatur im Vakuum getrocknet. Man erhält 595 g 4-Pentoxyphenylboronsäure-(2,2-dimethylpropan-1,3-diol)ester (4-n-Pentoxyphenylboronsäure-neopentylglykolester) vom Schmelzpunkt 80-82°.

### Herstellung von 4"-n-Pentoxy[1,1':4',1"]terphenyl-4-carbonsäure aus 4-n-Pentoxyphenylboronsäureestern

### Beispiel 5

### Herstellung von 4"-n-Pentoxy[1,1':4',1"]terphenyl-4-carbonsäure

162 g 4'-Iod[1,1']biphenyl-4-carbonsäure werden zusammen mit 129 g 4-n-Pentoxyphenyl-boronsäure-glykolester und 79,5 g Natriumcarbonat in 1,5 l Ethylenglykol vorgelegt, unter intensivem Rühren mit 350 mg PdCl₂(PPh₃)₂ versetzt und 6 Stunden bei 80°C gerührt. Man gießt die warme Reaktionsmischung vorsichtig auf eine Mischung aus 150 g 37%iger Schwefelsäure und 1000 g Wasser und erhitzt 30 Minuten auf 90-100°C. Nach Filtration und Wäsche mit Wasser wird das Rohprodukt bei 80°C/100 mbar getrocknet und im Anschluß aus Dimethylacetamid umkristallisiert. Dies liefert nach Trocknung 141 g (78%) 4"-n-Pentoxy[1,1':4',1"]-terphenyl-4-carbonsäure mit einer Reinheit >99%.

### Beispiel 6

### Herstellung von 4"-n-Pentoxy[1,1':4',1"]terphenyl-4-carbonsäure

32,4 g 4'-Iodbiphenyl-4-carbonsäure werden zusammen mit 25,8 g 4-n-Pentoxyphenyl-boronsäure-glykolester und 15,9 g Natriumcarbonat in 300 ml Ethylenglykol vorgelegt, unter intensivem Rühren mit 70 mg PdCl₂(PPh₃)₂ versetzt und 6 Stunden bei 120°C gerührt. Man gießt die warme Reaktionsmischung vorsichtig auf eine Mischung aus 30 g 37%iger Schwefelsäure und 200 g Wasser und erhitzt 30 Minuten auf 90-100°C. Nach Filtration und Wäsche mit Wasser wird das Rohprodukt bei 80°C/100 mbar getrocknet und im Anschluß daran aus Dimethylacetamid umkristallisiert. Dies liefert nach Trocknung 25,2 g (70 %) 4"-n-Pentoxy[1,1':4',1"]terphenyl-4-carbonsäure mit einer Reinheit >99 %.

### Beispiel 7

### Herstellung von 4"-n-Pentoxy[1,1':4',1"]terphenyl-4-carbonsäure

32,4 g 4'-Iod[1,1']biphenyl-4-carbonsäure werden zusammen mit 25,8 g 4-n-Pentoxyphenyl-boronsäure-glykolester und 15,9 g Natriumcarbonat in 300 ml Ethylenglykol vorgelegt, unter intensivem Rühren mit 18 mg PdCl₂ und 26,6 mg PPh₃ versetzt und 6 Stunden bei 80°C gerührt. Man gießt die warme Reaktionsmischung vorsichtig auf eine Mischung aus 30 g 37%iger Schwefelsäure und 200 g Wasser und erhitzt 30 Minuten auf 90-100°C. Nach Filtration und Wäsche mit Wasser wird das Rohprodukt bei 80°C/100 mbar getrocknet und im Anschluß daran aus Dimethylacetamid umkristallisiert. Dies liefert nach Trocknung 24,1 g (67%) 4"-n-Pentoxy[1,1':4',1"]terphenyl-4-carbonsäure mit einer Reinheit >99 %.

### Beispiel 8

### Herstellung von 4"-n-Pentoxy[1,1':4',1"]terphenyl-4-carbonsäure

162 g 4'-Iod[1,1']biphenyl-4-carbonsäure werden zusammen mit 129 g 4-n-Pentoxyphenyl-boronsäure-glykolester und 79,5 g Natriumcarbonat in 1,5 l Ethylenglykol vorgelegt, unter intensivem Rühren mit 17,5 ml einer Pd(dba)₂-Lösung versetzt und 6 Stunden bei 80°C gerührt. Man gießt die warme Reaktionsmischung vorsichtig auf eine Mischung aus 150 g 37%iger Schwefelsäure und 1000 g Wasser und erhitzt 30 Minuten auf 90-100°C. Nach Filtration und Wäsche mit Wasser wird das Rohprodukt bei 80°C/100 mbar getrocknet und im Anschluß daran aus Dimethylacetamid umkristallisiert. Dies liefert nach Trocknung 132 g (73 %) 4"-n-Pentoxyterphenyl-4-carbonsäure mit einer Reinheit >99 %.

### Herstellen der Pd(dba)₂-Lösung:

Unter Inertgas werden 1,47 g Natriumtetrachiorpailadat in 175 ml Ethylenglykol suspendiert, auf 60°C erhitzt, mit 3,65 g Dibenzylidenaceton (dba) versetzt und 15 Minuten bei 60°C gerührt. Hiemach versetzt man mit 7,5 g Natriumacetat und rührt weitere 60 Minuten bei Raumtemperatur. Die dunkel gefärbte Lösung wird so eingesetzt

### Beispiel 9

### Herstellung von 4"-n-Pentoxy[1,1':4',1"]terphenyl-4-carbonsäure

20,1 g 4'-Iod[1,1']biphenyl-4-carbonsäure, 13,1 g Natriumcarbonat und 21,4 g 4-n-Pentoxy-phenylboronsäure-glykolester werden in 260 g Dimethylsulfoxid (DMSO) vorgelegt, mit 160 mg PdCl₂(PPh₃)₂ versetzt und 2 Stunden bei 100-110°C erhitzt. Man filtriert bei 40°C vom Feststoff ab, wäscht mit Dimethylsulfoxid und suspendiert den Feststoff in 100 ml Wasser. Danach wird auf 80°C erhitzt und es werden tropfenweise innerhalb 1 Stunde 47 g 37%iger Schwefelsäure zugegeben. Man rührt weitere 30 Minuten bei 80°C, kühlt auf 40°C ab und filtriert. Nach Trocknen und Kristallisation aus Dimethylacetamid erhält man 18 g (81 %) 4"-n-Pentoxy[1,1':4',1"]terphenyl-4-carbonsäure mit einer Reinheit >99 %.

### Beispiel 10

### Herstellung von 4"-n-Pentoxy[1,1':4',1"]terphenyl-4-carbonsäure

32,4 g 4'-Iodbiphenyl-4-carbonsäure werden zusammen mit 25,8 g 4-n-Pentoxyphenyl-boronsäure-glykolester und 15,9 g Natriumcarbonat in 300 ml Methanol/Ethylenglykol 9:1 vorgelegt, unter intensivem Rühren mit 70 mg PdCl₂(PPh₃)₂ versetzt und 6 Stunden unter Rückfluß gerührt. Man gießt die warme Reaktionsmischung vorsichtig auf eine Mischung aus 30 g 37%iger Schwefelsäure und 200 g Wasser und erhitzt 30 Minuten auf 90-100°C. Nach Filtration und Wäsche mit Wasser wird das Rohprodukt bei 80°C/100 mbar getrocknet und im Anschluß daran aus Dimethylacetamid umkristallisiert. Dies liefert nach Trocknung 28,9 g (80 %) 4"-n-Pentoxy[1,1':4',1"]terphenyl-4-carbonsäure mit einer Reinheit >99 %.

### Beispiel 11

### Herstellung von 4"-n-Pentoxy[1,1':4',1"]terphenyl-4-carbonsäure

162 g 4'-Iod[1,1']biphenyl-4-carbonsäure werden zusammen mit 155 g 4-Pentoxyphenyl-boronsäure-(2,2-dimethylpropan-1,3-diol)ester und 79,5 g Natriumcarbonat in 1,5 l Ethylenglykol vorgelegt, unter intensivem Rühren mit 350 mg PdCl₂(PPh₃)₂ versetzt und 6 Stunden bei 80°C gerührt. Man gießt die warme Reaktionsmischung vorsichtig auf eine Mischung aus 150 g 37 %iger Schwefelsäure und 1000 g Wasser und erhitzt 30 Minuten auf 90-100°C. Nach Filtration und Wäsche mit Wasser wird das Rohprodukt bei 80°C/100 mbar getrocknet und im Anschluß aus Dimethylacetamid umkristallisiert. Dies liefert nach Trocknung 43,2 g (24 %) 4"-n-Pentoxyterphenyl-4-carbonsäure mit einer Reinheit >99 %.

### Beispiel 12

### Herstellung von 4"-n-Pentoxy[1,1':4',1"]terphenyl-4-carbonsäure aus 4-n-Pentoxyphenylboronsäure

34,1 g (0,1 mol) 4'-Iod[1,1']biphenyl-4-carbonsäure 95%ig werden zusammen mit 26 g (0,125 mol) 4-n-Pentoxyphenylboronsäure, 15,9 g (0,15 mol) Soda und 70 mg Bis(triphenylphosphin)palladiumdichlorid (PdCl₂(PPh₃)₂) in 300 ml DMSO vorgelegt.

Man rührt die Suspension 6 Stunden bei 80°C, filtriert den Feststoff ab, trägt in Wasser ein, säuert mit 37%iger Schwefelsäure an, erwärmt 30 Minuten auf 95°C und filtriert erneut. Nach Umkristallisation aus Dimethylformamid (DMF) erhält man 22,1 g (61 %) 4"-Pentoxy[1,1':4',1"]terphenyl-4-carbonsäure.

### Herstellung von 4"-n-Pentoxy[1,1':4',1"]terphenyl-4-carbonsäuremethylester aus 4'-Jod[1,1']biphenyl-4-carbonsäure-methylester

### Beispiel 13

### Herstellung von 4"-n-Pentoxy[1,1':4',1"]terphenyl-4-carbonsäuremethylester

33,8 g (0,1 mol) 4'-Iod[1,1']biphenyl-4-carbonsäuremethylester werden zusammen mit 29,3 g (0,125 mol) 4-n-Pentoxyphenylboronsäure-glykolester, 70 mg Bis(triphenyl-phosphin)palladiumdichiorid und 15,9 g (0,15 mol) Soda in 300 ml DMF vorgelegt und 12 Stunden bei 80°C gerührt. Man filtriert, wäscht mit Wasser und kristallisiert den getrockneten Rückstand aus DMF um. Dies liefert 20,5 g (45 %) 4"-n-Pentoxy-[1,1':4',1"]terphenyl-4-carbonsäure-methylester vom Schmp. 248°C.

### Vergleichsbeispiele gemäß WO 94/25050 zur Herstellung von 4"-n-Pentoxy[1,1':4',1"]terphenyl-4-carbonsäure

### Vergleichsbeispiel 1

### Herstellung von 4'-n-Pentoxy[1,1']biphenyl-4-boronsäure aus 4-Brom-4'-n-pentoxy[1,1']biphenyl

31,9 g (0,1 mol) 4-Brom-4'-n-pentoxy[1,1']biphenyl werden unter Stickstoffatmosphäre in 640 ml Tetrahydrofuran gelöst, auf -78°C gekühlt und innerhalb von 2 Stunden tropfenweise mit 67 ml (0,11 mol) einer 15%igen Lösung von n-Butyilithium in Hexan versetzt. Dabei wird die Innentemperatur in einem Bereich von -78°C bis -65°C gehalten. Nach beendeter Zugabe wird die dicke, milchige Suspension weitere 15 Minuten bei -78°C gerührt und im Anschluß daran mit 25,5 ml (0,11 mol) Triisopropylborat innerhalb 15 Minuten bei -78°C tropfenweise versetzt. Nach beendeter Boratzugabe erhält man eine klare Lösung, welche 15 Minuten bei -78°C nachgerührt wird. Im Anschluß daran wird das Kältebad entfernt und nach 40 Minuten die Lösung mit 100 ml 2N Salzsäure auf pH 2 gestellt. Die Phasen werden getrennt, die organische Phase mit Wasser und gesättigter Kochsalzlösung gewaschen und im Anschluß daran werden die Lösungsmittel unter Zusatz von 200 ml Wasser destillativ entfernt. Der ausgefallene Feststoff wird abfiltriert und getrocknet. Man erhält 25,8 g (91 %) 4'-n-Pentoxy[1,1']biphenyl-4-boronsäure vom Schmelzpunkt 148-150°C.

### Vergleichsbeispiel 2

### Herstellung von 4"-Pentoxy[1,1':4',1"]terphenyl-4-carbonsäure aus 4'-n-Pentoxy[1,1']biphenyl-4-boronsäure

25 g (0,088 mol) 4'-n-Pentoxy[1,1']biphenyl-4-boronsäure und 21,8 g (0,088 mol) 4-lodbenzoesäure werden unter Inertgasatmosphäre in einer Mischung aus 270 ml Ethanol, 750 ml Toluol und 132 ml einer 2M Sodalösung suspendiert, mit 5,08 g (4,4 mmol) Tetrakis(triphenylphosphin)palladium versetzt und im Anschluß daran 18 Stunden unter Rückfluß erhitzt.
Die grau-braune Mischung wird abgekühlt, angesäuert und mit Ethylacetat extrahiert. Die organische Phase wird mit Wasser und gesättigter Kochsalzlösung gewaschen, getrocknet (Natriumsulfat) und über Celite filtriert. Nach Entfernen der Lösungsmittel erhält man 1,2 g eines Feststoffes, der nach HPLC-Analyse (Vergleich mit Referenzsubstanz) jedoch keinerlei 4"-n-Pentoxy[1,1':4',1"]terphenyl-4-carbonsäure enthält.
Eine Bildung von 4-n-Pentoxy[1,1':4',1"]terphenyl-4-carbonsäure hat auf dem in WO 94/25050 angegebenen Syntheseweg offensichtlich nicht stattgefunden.

## Patentansprüche

1. Verfahren zur Herstellung von [1,1':4',1"]-Terphenylverbindungen der Formel 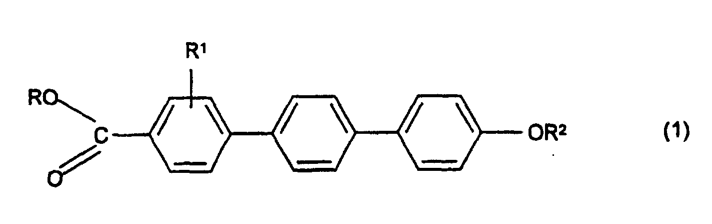 worin R Wasserstoff oder ein geradkettiger oder verzweigter (C₁-C₄)-Alkylrest, R¹ Wasserstoff, ein geradkettiger oder verzweigter (C₁-C₄)-Alkylrest oder ein geradkettiger oder verzweigter (C₁-C₄)-Alkoxyrest und R² Wasserstoff, ein geradkettiger (C₁-C₁₂)-Alkylrest, ein unsubstituierter Phenylrest, ein durch eine oder zwei (C₁-C₄)-Alkylgruppen oder (C₁-C₄)-Alkoxygruppen substituierter Phenylrest oder ein Rest -(CH₂)ₓOR³, worin x für eine ganze Zahl von 1 bis 4 und R³ für einen geradkettigen oder verzweigten (C₁-C₄)-Alkylrest steht, ist, **dadurch gekennzeichnet, daß** man ein Metallaryl der Formel , 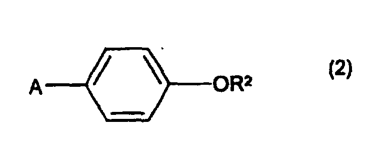 worin A für ein einwertiges Metall oder MeX, wobei Me ein zweiwertiges Metall und X Cl, Br oder J bedeutet, steht und R² für A oder einen trisubstituierten Silylrest steht oder die in Formel (1), ausgenommen Wasserstoff, angegebene Bedeutung hat, mit einem Borsäureester bei -80 bis 40 °C in Gegenwart eines inerten Lösungsmittels umsetzt, das Reaktionsprodukt durch Hydrolyse in eine Boronsäure der Formel 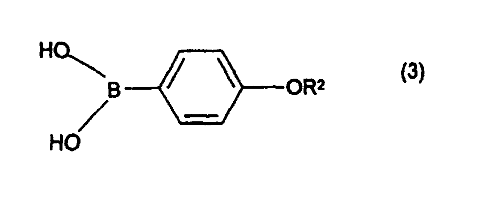 überführt, die Boronsäure, ein aus Boronsäure durch Abspalten von Wasser erhältliches Boronsäureanhydrid oder ein Gemisch aus Boronsäure und Boronsäureanhydrid mit einem Alkohol umsetzt, den dabei gebildeten Boronsäureester mit einer Biphenylverbindung der Formel 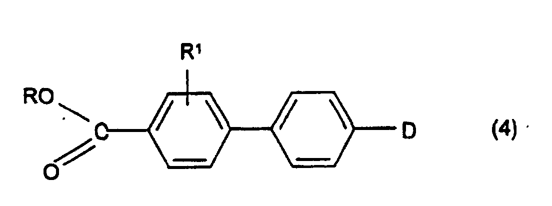 worin R und R¹ die in Formel (1) angegebene Bedeutung haben und D für Cl, Br, I, O₃S-CₙF₂ₙ₊₁, wobei n eine ganze Zahl von 1 bis 4 ist, oder für N₂⁺Y⁻, wobei Y⁻ ClO₄⁻, BF₄⁻ oder HSO₄⁻ ist, steht, bei 40 bis 180°C in Gegenwart eines Katalysators, eines säurebindenden Mittels und eines polaren Lösungsmittels umsetzt.

2. Verfahren nach Anspruch 1, **dadurch gekennzeichnet, daß** man ein Metallaryl der Formel (2), worin A für Li, Na, K, MgX oder ZnX und X für Cl, Br oder I steht, einsetzt.

3. Verfahren nach Anspruch 1 oder 2, **dadurch gekennzeichnet, daß** man ein Metallaryl der Formel (2), worin A für MgCl, MgBr oder MgI steht, einsetzt.

4. Verfahren nach einem oder mehreren der Ansprüche 1 bis 3, **dadurch gekennzeichnet, daß** man einen Borsäureester B(OR')₃, worin R' gleich oder voneinander verschieden ist und für einen geradkettigen oder verzweigten (C₁-C₈)-Alkylrest, einen unsubstituierten oder durch eine oder zwei (C₁-C₄)-Alkylgruppen oder (C₁-C₄)-Alkoxygruppen substituierten Phenylrest steht, einsetzt.

5. Verfahren nach einem oder mehreren der Ansprüche 1 bis 4, **dadurch gekennzeichnet, daß** man als inertes Lösungsmittel einen Dialkylether mit 1 bis 4 C-Atomen je Alkylrest, einen cycloaliphatischen Ether mit 4 oder 5 C-Atomen im Ring, ein Formaldehyd-dialkylacetal, einen 1,2-Dialkylglykoiether mit 1 bis 4 C-Atomen je Alkylrest, ein Gemisch derselben oder ein Gemisch derselben mit Toluol einsetzt.

6. Verfahren nach einem oder mehreren der Ansprüche 1 bis 5, **dadurch gekennzeichnet, daß** man als Alkohol einen (C₁-C₈)-Alkylalkohol, ein (C₂-C₆)-Alkandiol-1,2, ein (C₃-C₆)-Alkandiol-1,3, ein (C₄-C₆)-Alkandiol-1,4 oder 1,2-Dihydroxybenzol einsetzt.

7. Verfahren nach einem oder mehreren der Ansprüche 1 bis 5, **dadurch gekennzeichnet, daß** man die Boronsäure, das Boronsäureanhydrid oder das Gemisch aus Boronsäure und Boronsäureanhydrid anstelle des Boronsäureesters mit der Biphenylverbindung der Formel (4) umsetzt.

8. Verfahren nach einem oder mehreren der Ansprüche 1 bis 7, **dadurch gekennzeichnet, daß** man eine Biphenylverbindung der Formel (4), worin D für Cl, Br, I oder N₂⁺Y⁻ steht, einsetzt.

9. Verfahren nach einem oder mehreren der Ansprüche 1 bis 7, **dadurch gekennzeichnet, daß** man als Katalysator Palladium, eine Palladiumverbindung oder eine Nickelverbindung einsetzt.

10. Verfahren nach einem oder mehreren der Ansprüche 1 bis 9, **dadurch gekennzeichnet, daß** man als polares Lösungsmittel einen Alkohol, ein Sulfoxid, ein Sulfon, ein Amid und gegebenenfalls Wasser oder ein Gemisch derselben einsetzt.

11. Die Verbindungen 4-n-Pentoxyphenylboronsäure, trimeres 4-n-Pentoxyboronsäureanhydrid, 4-n-Pentoxyphenylboronsäure-glykolester und 4-n-Pentoxyphenylboronsäure-neopentylglykolester.

## Claims

1. A method for producing [1,1':4',1"]-terphenyl compounds of the formula 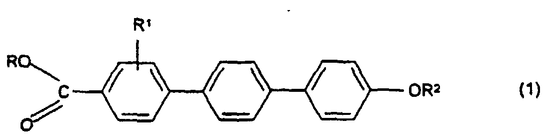 in which R is hydrogen or a straight-chain or branched C₁-C₄-alkyl radical, R¹ is hydrogen, a straight-chain or branched C₁-C₄-alkyl radical or a straight-chain or branched C₁-C₄-alkoxy radical and R² is hydrogen, a straight-chain C₁-C₁₂-alkyl radical, an unsubstituted phenyl radical, a phenyl radical which is substituted by one or two C₁-C₄-alkyl groups or C₁-C₄-alkoxy groups, or a radical -(CH₂)ₓOR³ in which x is an integer from 1 to 4 and R³ is a straight-chain or branched C₁-C₄-alkyl radical, which comprises reacting a metal aryl of the formula 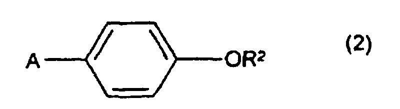 in which A is a monovalent metal or MeX, where Me is a divalent metal and X is Cl, Br or I, and R² is A or a trisubstituted silyl radical, or has the meaning indicated in formula (1), excepting hydrogen, with a boric ester at - 80 to 40°C in the presence of an inert solvent, converting the reaction product by hydrolysis into a boronic acid of the formula 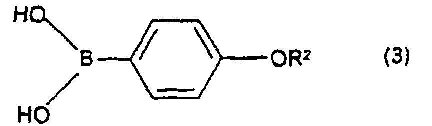 reacting the boronic acid, a boronic anhydride obtainable from boronic acid by elimination of water, or a mixture of boronic acid and boronic anhydride, with an alcohol, and reacting the boronic ester formed thereby with a biphenyl compound of the formula 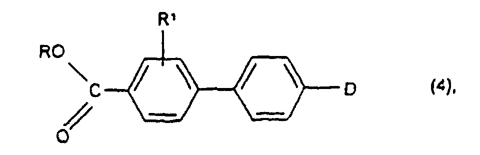 in which R and R¹ have the meaning indicated in formula (1), and D is Cl, Br, I, O₃S-CₙF₂ₙ₊₁, where n is an integer from 1 to 4, or N₂⁺Y⁻ where Y⁻ is ClO₄⁻, BF₄⁻ or HSO₄⁻, at 40 to 180°C in the presence of a catalyst, of an acid-binding agent and of a polar solvent.

2. The method as claimed in claim 1, wherein a metal aryl of the formula (2) in which A is Li, Na, K, MgX or ZnX and X is Cl, Br or I is employed.

3. The method as claimed in claim 1 or 2, wherein a metal aryl of the formula (2) in which A is MgCl, MgBr or Mgl is employed.

4. The method as claimed in one or more of claims 1 to 3, wherein a boric ester B(OR')₃ in which R' is identical to or different from one another and is a straight-chain or branched C₁-C₈-alkyl radical, or a phenyl radical which is unsubstituted or substituted by one or two C₁-C₄-alkyl groups or C₁-C₄-alkoxy groups is employed.

5. The method as claimed in one or more of claims 1 to 4, wherein a dialkyl ether having 1 to 4 carbon atoms in each alkyl radical, a cycloaliphatic ether having 4 or 5 carbon atoms in the ring, a formaldehyde dialkyl acetal, a 1,2-dialkyl glycol ether having 1 to 4 carbon atoms in each alkyl radical, a mixture thereof or a mixture thereof with toluene is employed as inert solvent.

6. The method as claimed in one or more of claims 1 to 5, wherein a C₁-C₈-alkyl alcohol, a C₂-C₆-alkane-1,2-diol, a C₃-C₆-alkane-1,3-diol, a C₄-C₆-alkane-1,4-diol or 1,2-dihydroxybenzene is employed as alcohol.

7. The method as claimed in one or more of claims 1 to 5, wherein the boronic acid, the boronic anhydride or the mixture of boronic acid and boronic anhydride is reacted in place of the boronic ester with the biphenyl compound of the formula (4).

8. The method as claimed in one or more of claims 1 to 7, wherein a biphenyl compound of the formula (4) in which D is Cl, Br, I or N₂⁺Y⁻ is employed.

9. The method as claimed in one or more of claims 1 to 7, wherein palladium, a palladium compound or a nickel compound is employed as catalyst.

10. The method as claimed in one or more of claims 1 to 9, wherein an alcohol, a sulfoxide, a sulfone, an amide and, where appropriate, water or a mixture thereof is employed as polar solvent.

11. The compound 4-n-pentoxyphenylboronic acid, trimeric 4-n-pentoxyphenylboronic anhydride, glycol ester of 4-n-pentoxyphenylboronic acid or neopentyl glycol ester of 4-n-pentoxyphenylboronic acid.

## Revendications

1. Procédé de préparation de [1,1':4',1"]-terphényles de formule 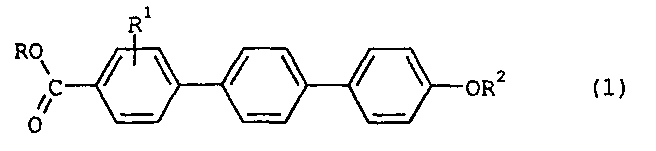 dans laquelle R est un atome d'hydrogène ou un radical alkyle en C₁-C₄ à chaîne droite ou ramifiée, R¹ est un atome d'hydrogène, un radical alkyle en C₁-C₄ à chaîne droite ou ramifiée, ou un radical alcoxy en C₁-C₄ à chaîne droite ou ramifiée, et R² est un atome d'hydrogène, un radical en C₁-C₁₂ à chaîne droite, un radical phényle non substitué, un radical phényle substitué par un ou deux groupes alkyle en C₁-C₄ ou alcoxy en C₁-C₄, ou encore un radical - (CH₂)ₓOR³ dans lequel x est un nombre entier de 1 à 4, et R³ est un radical alkyle en C₁-C₄ à chaîne droite ou ramifiée, **caractérisé en ce qu'**on fait réagir un arylmétal de formule 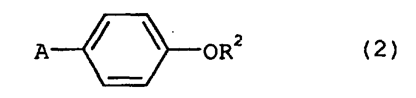 dans laquelle A est un métal monovalent ou MeX, Me étant un métal divalent et X représente en Cl, Br ou I, et R² est A ou un radical silyle trisubstitué, ou encore a les significations données pour la formule (1), à l'exception de l'hydrogène, avec un ester de l'acide borique à une température de -80 à 40°C en présence d'un solvant inerte, on convertit le produit de réaction par hydrolyse en un acide boronique de formule 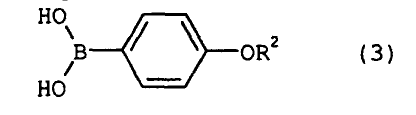 on fait réagir avec un alcool l'acide boronique, un anhydride boronique pouvant être obtenu à partir d'acide boronique par élimination d'eau ou un mélange d'acide boronique et d'anhydride boronique, on fait réagir à une température de 40 à 180°C, en présence d'un catalyseur, d'un agent fixant les acides et d'un solvant polaire, l'ester de l'acide boronique ainsi formé, avec un biphénile de formule 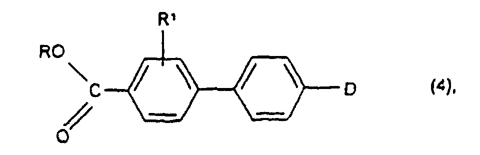 dans laquelle R et R¹ ont les significations données pour la formule (1), et D est Cl, Br, I, O₃S-CₙF₂ₙ₊₁, , étant un nombre entier de 1 à 4, ou encore N₂⁺Y⁻, Y⁻ représentant ClO₄⁻, BF₄⁻ ou HSO₄⁻.

2. Procédé selon la revendication 1, **caractérisé en ce qu'**on utilise un arylmétal de formule (2) dans laquelle **A** est Li, Na, K, MgX ou ZnX, et X est Cl, Br ou I.

3. Procédé selon la revendication 1 ou 2, **caractérisé en ce qu'**on utilise un arylmétal de formule (2) dans laquelle **A** est MgCl, MgBr ou MgI.

4. Procédé selon l'une ou plusieurs des revendications 1 à 3, **caractérisé en ce qu'**on utilise un ester de l'acide borique B(OR')₃, où les radicaux R' sont identiques les uns aux autres ou différents les uns des autres et représentent chacun un radical alkyle en C₁-C₈ à chaîne droite ou ramifiée ou un radical phényle non substitué ou substitué par un ou deux groupes alkyle en C₁-C₄ ou alcoxy en C₁-C₄.

5. Procédé selon l'une ou plusieurs des revendications 1 à 4, **caractérisé en ce qu'**on utilise en tant que solvant inerte un dialkyléther ayant 1 à 4 atomes de carbone dans chaque fragment alkyle, un éther cycloaliphatique ayant 4 à 5 atomes de carbone dans le noyaun un acétal dialkylique du formaldéhyde, un éther du 1,2-dialkylglycol ayant 1 à 4 atomes de carnone par fragment alkyle, un mélange de caux-ci, ou un mélange de ceux-ci avec le toluène.

6. Procédé selon l'une ou plusieurs des revendications 1 à 5, **caractérisé en ce qu'**on utilise en tant qu'alcool un alcool alkylique en C₁-C₈, un alcanediol-1,2 en C₂-C₆, un alcanediol-1,3 en C₃-C₆, un alcanediol-1,4 en C₄-C₆ ou le 1,2-dihydroxybenzène.

7. Procédé selon l'une ou plusieurs des revendications 1 à 5, **caractérisé en ce qu'**on fait réagir avec le biphényle de formule (4) l'acide boronique, l'anhydride boronique ou le mélange d'acide boronique et d'anhydride boronique à la place de l'ester boronique.

8. Procédé selon l'une ou plusieurs des revendications 1 à 7, **caractérisé en ce qu'**on utilise un biphényle de formule (4) dans laquelle D est Cl, Br, I ou N₂⁺Y⁻.

9. Procédé selon l'une ou plusieurs des revendications 1 à 7, **caractérisé en ce qu'**on utilise en tant que catalyseur le palladium, un composé du palladium ou un composé du nickel.

10. Procédé selon l'une ou plusieurs des revendications 1 à 9, **caractérisé en ce qu'**on utilise en tant que solvant polaire un alcool, un sulfoxyde, une sulfone, un amide et éventuellement de l'eau ou un mélange de ceux-ci.

11. Les composés acide 4-n-pentoxyphénylboronique, anhydride 4-n-pentoxyboronique trimère, ester du glycol de l'acide 4-n-pentoxyphénylboronique et ester du néopentylglycol de l'acide 4-n-pentoxyphénylboronique.
